(19) Europäisches Patentamt

European Patent Office

Office européen des brevets

(11) **EP 1 459 761 A1**

(12) **EUROPEAN PATENT APPLICATION**
published in accordance with Art. 158(3) EPC

(43) Date of publication:
**22.09.2004 Bulletin 2004/39**

(51) Int Cl.7: **A61K 39/395**, A61P 35/00

(21) Application number: **01969178.1**

(22) Date of filing: **08.06.2001**

(86) International application number:
**PCT/CN2001/000924**

(87) International publication number:
**WO 2002/100431 (19.12.2002 Gazette 2002/51)**

(84) Designated Contracting States:
**AT BE CH CY DE DK ES FI FR GB GR IE IT LI LU MC NL PT SE TR**
Designated Extension States:
**AL LT LV MK RO SI**

(71) Applicants:
• **The Fourth Military Medical University Shanxi Province 710038 P.R. (CN)**

• **Hao, Xiaoke Shanxi Province 710038 P.R. (CN)**

(72) Inventor: **HAO, Xiaoke, Tangdu Hospital Shanxi Province 710038 P.R. (CN)**

(74) Representative: **Warcoin, Jacques et al Cabinet Régimbeau, 20, rue de Chazelles 75847 Paris Cedex 17 (FR)**

(54) **A PHARMACEUTICAL KIT COMPRISING ANTI-HUMAN SEMINAL PLASMA PROTEIN SINGLE CHAIN ANTIBODY/HUMAN CARBOXYPEPTIDASE FUSION PROTEIN AND PRODRUG**

(57) This invention is in the field of antibody-directed enzyme prodrug therapy (ADEPT). More particularly, the invention relates to a kit comprising an anti-human γ-seminoprotein single-chain antibody (γ-Sm scFv)/human carboxypeptidase A(hCPA) fusion protein and a prodrug which is a methotrexate-α-peptide. The kit is useful in prostate cancer treatment with the ADEPT. The invention also relates to an anti-γ-seminoprotein single-chain antibody (γ-Sm scFv)/human carboxypeptidase (hCPA) fusion protein.

**Description**

**FIELD OF INVENTION**

This invention is in the field of antibody-directed enzyme prodrug therapy (ADEPT). More particularly, the invention relates to a kit comprising an anti-human γ-seminoprotein single-chain antibody (γ-Sm scFv)/human carboxypeptidase A(hCPA) fusion protein and a prodrug which is a methotrexate-α-peptide. The kit is useful in prostate cancer treatment with the ADEPT. The invention also relates to an anti-γ-seminoprotein single-chain antibody (γ-Sm scFv)/ human carboxypeptidase (hCPA) fusion protein.

**BACKGROUND OF INVENTION**

Highly selective tumor chemotherapy is one of the important topics for tumor research at present. Most chemotherapeutic drugs in use are far from being highly selective towards tumor tissues. What is more, the drugs are toxic to normal tissues while taking effect in tumor tissues. Monoclonal antibodies have provided a foundation for selectively directed therapy of tumors. Despite the expectation that by means of monoclonal antibodies cytotoxic drugs can be directly carried towards tumor tissues, the toxic drugs cannot be concentrated within tumor sites as effectively as they are expected. This is due to several factors: 1) one antibody molecule can carry only a small amount of drug; 2) antigens in tumor tissues vary in their numbers; 3) it is relatively difficult for big antibody-drug complexes to access substantial tumor locations; 4) a host immune reaction to extrinsic antibodies may be stimulated. It was in such a situation, the antibody-directed enzyme prodrug therapy (ADEPT) proposed by Bagshawoe (Br J Cancer, 1987, 56(5): 531-532) aroused widespread concern in the field of chemotherapy. The strategy of the ADEPT is: by using a monoclonal antibody as a carrier, an enzyme which is specific for activating a prodrug (called activating enzyme) is selectively directed to a tumor site where it catalyzes the prodrug into activated cytotoxic molecules. This scenario can overcome many problems associated with chemical conjugates and immune-toxins, since only a tiny amount of enzyme-antibody cross-linked complex is sufficient to convert a great lot of prodrug into activated toxic drug in the tumor site. In the first ADEPT proposed by Bagshawoe in 1987, a carboxypeptidase G2 gene was isolated from *Bacillus pyocyaneus* and cloned into *E. coli.* The enzyme thus produced in *E. coli* was initially employed to hydrolyze methtrexate and later was used to activate chlorethazine benzoate derivatives via removing their glutamic acid moiety. This is the most representative ADEPT strategy and has entered clinical trial on a small scale. The advantage of the strategy lies in that one enzyme molecule is able to convert many prodrug molecules. For example, one mol of carboxypeptidase G2 can degrade 800 mol of chlorethazine benzoate substrate in just one second and produce high concentration of active drug at tumor sites. This can compensate the lower affinity of immuno-conjugates in clinical applications. It has also been proved that high-concentration of drugs concentrated on the surface of tumor cells are more effective than the same concentration of active drugs which are systematically administered.

Methotrexate (MTX) is an anti-metabolic drug that is widely used in clinical chemotherapy. However, it can inhibit normal cells while killing tumor cells and thus results in a serious toxic reaction. This has limited the clinical application of MTX at high concentration and large dose. As a result, the therapeutic effect of MTX is hindered. If an amino acid is linked at the α-carboxyl of the glutamate at the carboxyl terminal of MTX, MTX is converted to an MTX-α-peptide which has no toxicity to any cells. This compound can release cytotoxic MTX when the peptide bond at the carboxyl terminal is hydrolyzed by an appropriate carboxypeptidase. This result is desirable for the ADEPT strategy.

There have been many rapid developments in the research and application of the ADEPT since it was first introduced by Bagshawoe in 1987. Some prodrugs have already entered Phase I clinical trial, however, the biggest obstacle to the *in vivo* application of the ADEPT goes to the heterology of a murine monoclonal antibody and/or enzyme. Therefore, it is necessary to prepare a fusion protein of a humanized antibody/human activating enzyme for better effects of the ADEPT. Heterology of antibody may be avoided by replacing a murine monoclonal antibody with an ScFv because ScFv only retains the variable region of an antibody that is responsible for the binding specificity of an antibody, and eliminates the constant region that has great diversity. ScFv takes up only 1/6 of an integral antibody and has good affinity and stability. The fusion protein of an ScFv and an activating enzyme is able to both bind specifically to tumor cells and hydrolyze the prodrug, with a dominant advantage that it can be prepared on a large scale and favorable for clinical application.

Gama-Seminoprotein (γ-Sm) is a prostate-specific antigen secreted by prostate epithelium into semen, with a molecular weight of 23, 000-33,000. In the past it was used in forensic identification. Recently, by using histochemical staining method, it was found that γ-Sm is localized in both prostate cancer and their metastatic cells but not in other human tissues and malignant tumors. Just like other prostate-specific antigens (PSA), γ-Sm has been recognized as a specific marker for prostate cancer.

Carboxypeptidase A (CP-A) is a carboxypeptidase secreted by some mammal tissues. The gene of CP-A has a total length of 1251bp and encodes 417 amino acids. This enzyme can hydrolyze aliphatic and aromatic amino acids from the C terminal of a polypeptide successively. With this property, CP-A is the first enzyme that has been used in the ADEPT research.

In view of above, starting from the anti-γ-seminoprotein single-chain antibody (γ-Sm scFv) which was constructed and expressed previously, the inventor has done further study on the treatment of prostate cancer using CP-A and methotrexate-α-peptides and finally accomplished the invention.

Thus, it is an object of the invention to produce a pharmaceutical kit for the treatment of prostate cancer with the antibody-directed enzyme prodrug therapy (ADEPT). The kit comprises a fusion protein of anti-γ-seminoprotein single-chain antibody/human carboxypeptidase A, and a prodrug which is a methotrexate-α-peptide.

Another object of the invention is to provide a fusion protein of anti-γ-seminoprotein single-chain antibody/ human carboxypeptidase A (anti-γ-Sm scFv/hCPA) as well as a polynucleotide encoding the fusion protein.

## SUMMARY OF THE INVENTION

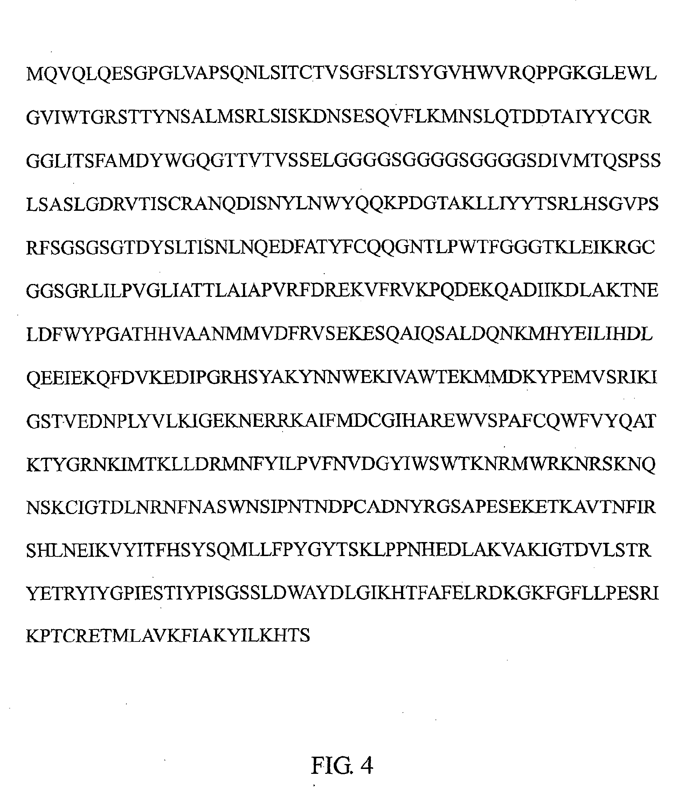

In one aspect, the invention provides a pharmaceutical kit for the treatment of prostate cancer with the ADEPT. The kit comprises several containers which separately contains a fusion protein of an anti-γ-seminoprotein single-chain antibody (γ-Sm scFv)/human carboxypeptidase A(hCPA), a prodrug which is a methotrexate-α-peptide and pharmacologically acceptable adjuvants. The fusion protein of γ-Sm scFv/hCPA should be administered at least 72 hours before the prodrug is administered. Preferably, the prodrug is methotrexate-α-phenylalanine. The amino acid sequence of the γ-Sm scFv/hCPA fusion protein is shown in FIG.4.

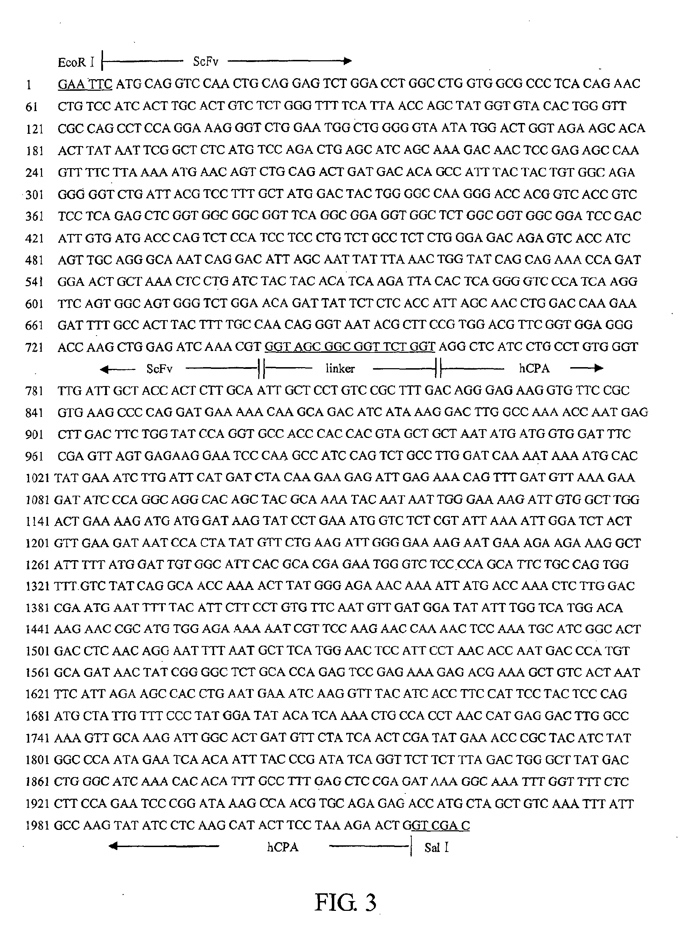

In another aspect, the invention provides a fusion protein of γ-Sm scFv/hCPA as well as a polynucleotide encoding the fusion protein. The amino acid sequence of the fusion protein is shown in FIG. 4, while the polynucleotide sequence is shown in FIG.3.

## DETAILED DESCRIPTION OF THE INVENTION

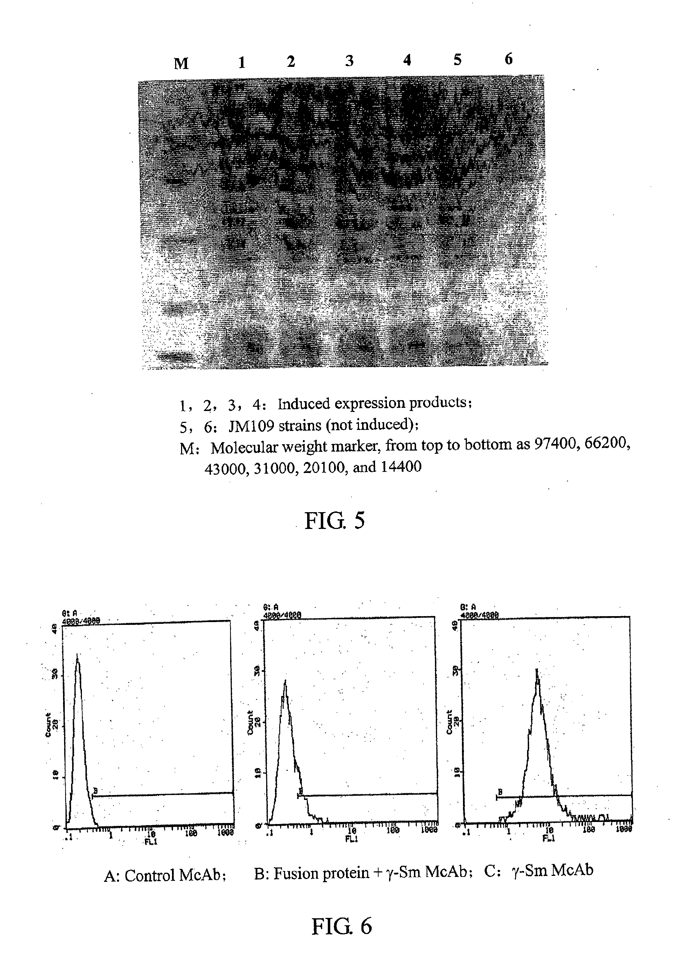

To obtain the fusion protein of the invention, initially an anti-γ-seminoprotein single-chain antibody (γ-Sm scFv) is constructed starting from a hybridoma strain $E_4B_7$ (kept by the inventor). The gene for human carboxypeptidase may be amplified from human pulmonary tissues by RT-PCR. Next, a fusion gene encoding the fusion protein of γ-Sm scFv/hCPA is constructed as described in the Examples. To allow the ScFv and hCPA in the fusion protein to form correct spatial structures and to keep their own biological functions, a linker encoding 6 amino acids is inserted between the coding regions of ScFv and hCPA using add-on PCR and gene splicing by overlap extension. Compared with conventional recombinant DNA techniques, this overlap extension technique does not use restriction endonuclease and ligase, so that it can avoid introducing nucleic acid sequence for the restriction endonuclease sites, as well as can precisely link the two gene fragments. Biological activity assay confirms that the expressed fusion protein has activities of both ScFv and hCPA (see in FIGS. 5 and 6).

Well-established human prostate cancer cell lines PC-3 (low metastasic) and PC-3m (high metastasic) and prostate cancer carrier BALB/C nude mice inoculated with PC-3m have been used as models to test the kit of this invention. The results indicate that this kit is significantly effective for prostate cancer. To optimize the effects of the therapy, the fusion protein in the kit should be administered at least 72 hours before the prodrug is administered. It is contemplated that the dosage and administration route may vary according to the judgment of skilled persons in the art and clinical doctors as well as the patient's individual condition.

The invention will be described in more detail with reference to the following examples and figures, wherein:

FIG. 1 shows the construction strategy of the fusion gene for anti-γ-seminoprotein single-chain antibody/human carboxypeptidase A.

FIG. 2 is the agarose gel electrophoresis result of the fusion gene of the invention.

FIG. 3 depicts the nucleotide sequence of the fusion gene of the invention.

FIG. 4 depicts the amino acid sequence of the fusion protein of the invention.

FIG. 5 shows the expression of the fusion protein of the invention.

FIG. 6 shows the binding activity of the fusion protein with PC cell lines.

FIG. 7 shows the hydrolytic activity of the fusion protein towards a prodrug, MTX-α-Phe.

FIG. 8 is a killing curve of the prodrug on PC cells in an experiment as performed in Example 2.

FIG. 9 shows the effects of various groups of drugs on the cell cycle of the PC cells. The experiment is described in Example 2.

FIG. 10 is the tumor growth curves of different treatment groups as indicated in Example 2.

FIG. 11 shows the tumor growth of the nude mice in the ADEPT treatment group and control group. The experiment is described in Example 2.

**Example 1: Preparation and expression of gene for $\gamma$-Sm scFv/hCPA fusion protein**

In this example, upon the construction of anti-$\gamma$-Sm scFv cDNA and amplification ofhCPA gene, anti-$\gamma$-Sm scFv/hCPA fusion gene was constructed using add-on PCR and gene splicing by overlap extension (SOE). The prokaryotic expression of the fusion gene and activities of the fusion protein were also analyzed.

**Materials and Methods**

**1. Materials**

Hybridoma cell line $E_4B_7$ which secrets anti-$\gamma$-Sm-McAb was prepared by the inventor previously. *E. coli* JM109 cell and pUC19 plasmid were purchased. Taq DNA polymerase and restriction endonuclease, (Reverse Transcription System), plasmid extraction and purification kit (Wizard™ Plus Minipreps DNA Purification System) and GST fusion protein expression vector pGEX-4T-1 were purchased from Promega Company. Rapid Ligation Kit was purchased from Boeringer Mannheim Company. Advantage™ PCR-Pure Kit was purchased from Clontech Company. IPTG was purchased from Huamei Company, Beijing China. The rest of the reagents were all products of Sigma.

**2. Construction, cloning and sequence analysis of anti-$\gamma$-Sm $E_4B_7$ ScFv gene**

**Total RNA extraction and reverse transcription:** Total RNA extraction was performed by conventional method. The total RNA extracted from $2\times10^7$ $E_4B_7$ cells was dissolved in 30µl of RNase-free water. 5µl of the total RNA was used in a reverse transcription reaction according to the manual provided by the manufacturer (Promega, USA).
**Amplification, cloning, identification and sequence analysis of genes coding for $E_4B_7$ McAb variable regions:** The following primers were used in the amplification of genes coding for variable regions of light and heavy chains:

$V_H$Back     5'-AGGT(CG) (AC)A(AG)CTGCAG(CG)AGTC(AT)GG-3'

$V_H$For     5'-TGAGGAAACGGTGACCGTGGTCCCTTGGCCCAG-3'

$V_L$Back     5'-GTGAATTCGACATCGTGATGACCCAGTCTCC-3'

$V_L$For     5'-CAGTCGACTAACGTTTGATCTCCAGCTTGGTCCC-3'

Using 5µl of the synthesized cDNA as template, $V_H$ gene was amplified with $V_H$Back and $V_H$For primers, while $V_L$ gene was amplified with $V_L$Back and $V_L$For primers respectively. The reactions were each performed for 30 cycles of 94°C for 60s, 55°C for 90s, and 72°C for 120s. 5µl of the product was taken from each reaction mixture and analyzed by 1.5% agarose gel electrophoresis. The remaining product was recovered with Advantage ™ PCR-Pure Kit. 2µl of the retrieval was used in a second round of PCR reaction. The PCR products were blunted by Klenow polymerase, after which $V_H$ gene was digested by Pst I, and $V_L$ gene was digested by EcoRI and Sel I. The digested products were ligated respectively with linearlized pUC19 plasmid using Rapid DNA Ligation Kit and transformed into competent E. coli JM109 cells. White colonies were selected by X-gal and IPTG. Recombinant plasmid DNA was extracted using Wizard™ Plus Minipreps DNA purification System and identified by restriction enzyme digestion. The recombinant pUC plasmids containing the gene fragment of interest were sequenced using PE373-A DNA Sequence Analyzer.

**Construction, cloning and sequence analysis of ScFv gene:** A 15-mer peptide $(Gly_4Ser)_3$ was used as a linker sequence linking the variable regions of the antibody. According to the linker sequence, two primers were designed as follows:

> 5'-GAGCTCGGTGGCGGCGGTTCAGGCGGAGGTGGCTCTGGCGGTGGCGGA-3'

> 5'-GGATCCGCCACCGCCAGAGCCACCTCCGCCTGAACCGCCGCCACCGAG-3'

A linker sequence containing Sac I and BamH I cohesive ends was formed after denaturing at 94°C for 10 min and annealing at 50°C for 7 min. Recombinant pUC19-Linker plasmid was constructed by ligating the linker sequence with pUC19 plasmid linearlized by Sac I and BamH I, and confirmed by sequence analysis after transforming into a host cell and being extracted therefrom.

Primers were designed according to the $V_H$ and $V_L$ sequences so as to allow, after PCR reactions, the restriction sites in the heavy chain being a 5' terminal EcoR I and a 3' terminal Sac I, and the restriction sites in the light chain being a 5' terminal BamH I and a 3' terminal Sal I. The amplified $V_H$ and $V_L$ genes were then cloned into the pUC19-Linker plasmid successively and allowed to express. A single-chain antibody was thus constructed. After transformation and selection, recombinant plasmid DNAs extracted from positive colonies were sequenced for confirmation.

### 3. Cloning and sequence analysis of hCPA gene

**Primer design and synthesis:** According to the complete sequence ofhCPA mRNA from Genbank, a pair of primers was designed flanking the coding region of the mature protein. The primers were synthesized by Beijing Saibaisheng Biotech Co. (Beijing, China), the sequences of which were as follows:

> 5' primer: 5'-TC<u>GAATTC</u>ATGAGGCTCATCCTGCCTGTGGGTTT-3';
>
>        EcoRI

> 3' primer: 5'-GAT<u>GTCGAC</u>CAGTTCTTTAGGAAGTATGCTTGAG-3';
>
>        Sal I

**Amplification of hCPA gene by RT-PCR:** 50mg of normal pulmonary tissues were taken from surgical lung cancer patient, from which total RNA was isolated by AGPC one-step extraction method. The RNA was then reversely transcribed by Reverse Transcription System according the manual (Promega, USA). PCR was performed using 2μl of the cDNA product as a template. The PCR reaction system was composed of 100 pmol of each primer, 8μl of 2.5mmol/LdNTPs, 6μl of 25mmol/L $MgCl_2$, 5μl of 10×PCR buffer and 5μl of Taq DNA polymerase. The total reaction volume was adjusted to 100μl by adding sterilized de-ionized water. The conditions for the PCR reaction included 30 cycles of denaturation at 94°C for 1 min, annealing at 50°C for 1.5 min, extension at 72°C for 2 min, and a final extension at 72°C for 10 min. The PCR product was analyzed with 15g/L agarose gel electrophoresis.

**Cloning and Sequencing of hCPA:** The PCR product of hCPA obtained in above step was cloned into plasmid pUC19 and positive colonies were identified by EcoRI and SalI digestion. Positive strains comprising hCPA recombinant plasmid were cultured and plasmid DNAs were extracted with Wizard™ Plus Minipreps DNA Purification System (Promega, USA). DNA sequence analysis was conducted using PE373-A DNA Sequence Analyzer.

### 4. Construction, cloning and sequencing of γ-Sm scFv/hCPA fusion gene

**Design of splicing primer:** Based upon the nucleotide sequences of γ-Sm scFv, hCPA and the linker, primers for the amplification and splicing of ScFv/hCPA gene were designed. All primers were synthesized by Saibaisheng Biotech Co. (Beijing, China). The sequences of the primers were as follows:

ScFv Back： 5'-GC<u>GAATTC</u>ATGCAGGTCCAACTGCAGGA-3';

EcoRI

hCPA For： identical to the hCPA 3' primer;

←ScFv For

3'-GTTCGACCTCTAGTTTGCACCATCGCCGCCAAGA-5'

5'-AGCGGCGGTTCTGGTAGGCTCATCCTGCCTGTG-3'

hCPA Back→

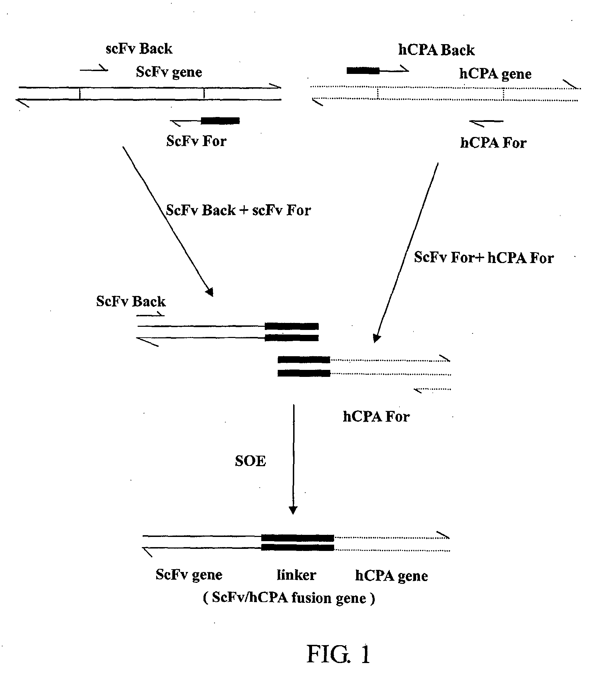

**Add-on PCR and overlap extension:** Add-on PCR reaction was performed on the ScFv and hCPA genes respectively. The products were spliced into anti-$\gamma$-Sm scFv/hCPA fusion gene using splicing of overlap extension (SOE) method (FIG. 1). The conditions for the reaction included 30-35 cycles of denaturation at 94°C for 1min, annealing at 50°C for 1min, and extension at 72°C for 1.5min.

**Cloning and sequencing of anti-$\gamma$-Sm ScFv/hCPA fusion gene:** Recombinant anti-$\gamma$-Sm ScFv/hCPA fusion gene was recovered and digested with EcoRI and SalI. The digested product was ligated with plasmid pUC19 linearlized by same enzymes and transformed into competent cells of JM109. Positive colonies were screened and plasmid DNAs extracted. The fusion gene was sequenced using an automatic fluorescent DNA sequencer.

**Expression of anti-$\gamma$-Sm ScFv/hCPA fusion protein:** After the sequence was determined, the fusion gene was cloned into pGEx-4T-1 vector and in turn the recombinant vector was transformed into competent cells of *E. coli* JM109. Protein expression was then induced with IPTG at a final concentration of 0.1mM for 5h. The recombinant protein was analyzed on a SDS polyacrylamide gel (SDS-PAGE). The protein bands were scanned using a Dual-wavelength Flying-Spot Scanner CS-9000 manufactured by Shimadzu Co. LTD. (Japan).

**Affinity and enzyme activity of anti-$\gamma$-Sm ScFv/hCPA fusion protein:** 100ml of the induced culture was taken and purified and denaturized by routine methods. Then the products were further purified by GST affinity chromatography and digested with 0.2% thrombin. The products were lyophilized and stored until use. The antibody affinity was determined by a competitive binding inhibition assay using flow cytometry. The fusion protein was added to a culture containing $1 \times 10^6$ PC-3 cell line and incubated for 45 min. Then a parent antibody ($\gamma$-Sm MAb) was added and incubated for 45 min. After fluorescein-labelled goat anti-mouse antibody was added, percentage and fluorescent intensity of the labeled fluorescent cell were determined with flow cytometry. Meanwhile positive control using parent antibody and control using an uncorrelated antibody were established. The enzyme activity was determined by HPLC.

**Results**

**1. Construction, cloning and sequence analysis of anti-$\gamma$-Sm $E_4B_7$ ScFv gene**

Primers $V_H$Back, $V_H$For, $V_L$Back and $V_L$For were designed according to the 5' terminus and 3' J region conservative sequence of the variable region of the murine antibody. $E_4B_7V_H$ and $V_L$ genes were amplified from $E_4B_7$ hybridoma cells. 360bp and 330bp DNA fragments were obtained after respective EcoR I and HindIII digestion, which were consistent with the expected size of the variable region of the murine antibody. An automatic fluorescent DNA sequencer was used for the sequencing of $E_rB_7V_H$ and $V_L$ genes. As a result, $V_H$ gene was an open reading frame 358bp in length and encoded 119 amino acids, with the amino acids at 22 and 95 being cysteine residues characteristic of an antibody variable region. $V_L$ gene was also an open reading frame made up of 326bp and encoded 108 amino acids with the amino acids at positions 23 and 88 being cysteine residues. PCR amplification and identification of the ligated product of $E_4B_7V_H$ and $V_L$ genes indicated a fragment of 750bp. An automatic fluorescent DNA sequencer was employed to sequence the product. The result indicated that the sequences of the $V_H$ and $V_L$ genes and the linker were all correct.

## 2. Amplification, cloning and sequencing of hCPA gene

The cDNA for human carboxypeptidase A was amplified from healthy human pulmonary tissues. A specific band of 1250bp was observed during 15g/L agarose gel electrophoresis analysis, which was consistent with its predicted molecular weight. The recombinant plasmid of pUC19/CPA was transformed into competent cells, and positive colonies were selected randomly to extract plasmid DNA for restriction confirmation. The sequence of the hCPA gene was determined using an automatic fluorescent DNA sequencer, which revealed that the nucleotide sequence and amino acid sequence deduced therefrom were consistent with the reported sequences previously.

## 3. Add-on PCR and overlap extension

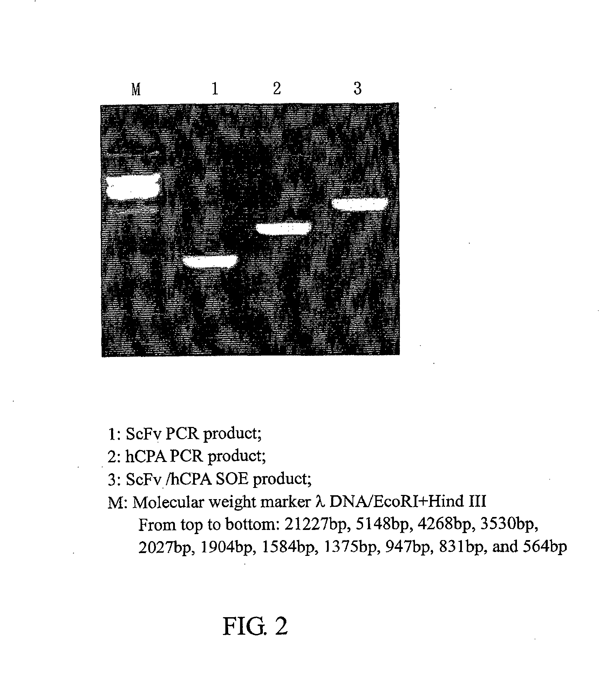

Add-on PCR reaction was performed to add complementary nucleotides on the 3' terminus of the anti-γ-Sm scFv gene and the 5' terminus of the hCPA gene respectively. Then, the above two PCR products were mixed and ligated as anti-γ-Sm scFv/hCPA fusion gene via SOE reaction. 15g/L agarose gel electrophoresis showed the fusion gene was approximately 1980 bp, which was consistent with the expected size (FIG. 2).

## 4. Sequence analysis of the anti-γ-Sm scFv/hCPA fusion gene

A positive clone was selected randomly to perform sequencing analysis. An automatic fluorescent DNA sequencer was employed for the sequencing. The result indicated that the sequences of anti-γ-ScFV and hCPA genes were consistent with those reported previously. The fusion gene, just as designed, have EcoRI and SalI sites, start codon and stop codon. There is an 18 bp linker sequence between the ScFv gene and hCPA gene, encoding an amino acid sequence of GSGGSG. FIG. 3 shows the nucleotide sequence of the fusion gene.

## 5. Expression of the anti-γ -Sm scFv/hCPA fusion protein

The expressed GST-ScFV/hCPA was analyzed by SDS-PAGE. The result indicated that there was a newly-emerged protein band with a *Mr* of 94,000 Dalton (FIG. 5). Thin-layer scanning found its expression accounting for 34% of the total proteins. The fusion protein appeared to be in the form of inclusion body. Purified ScFv/hCPA was obtained by routine denaturation, renaturation and purification by affinity chromatography. FIG. 4 shows the amino acid sequence of the fusion protein.

## 6. Affinity and enzyme activity of the fusion protein

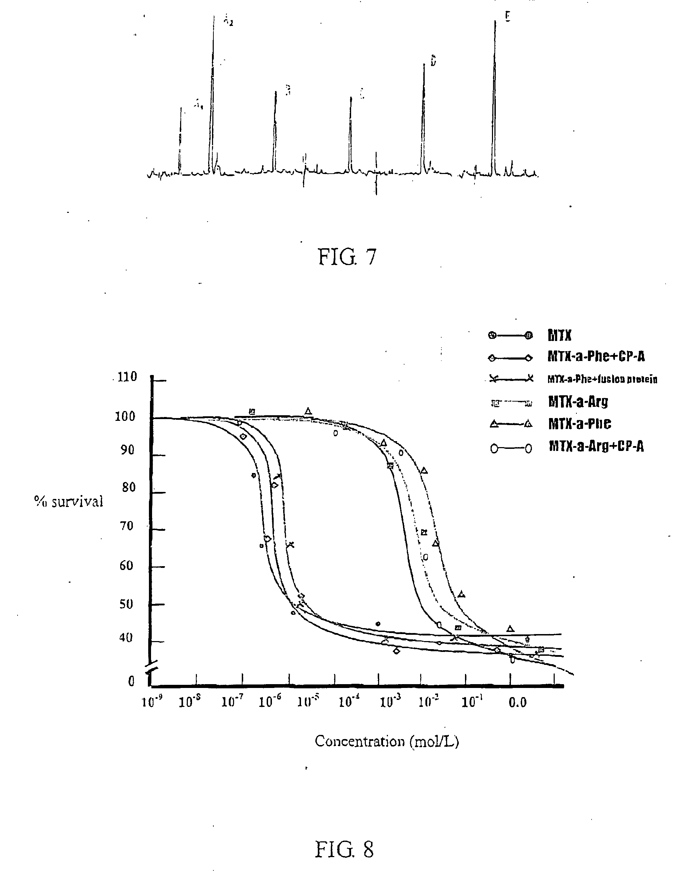

The results of competitive binding inhibition assay confirmed that this ScFv/hCPA fusion protein was capable of binding to human prostate cell lines PC-3 and PC-3m (FIG6). HPLC analysis indicated that the fusion protein can completely hydrolyze the prodrug MTX-α-Phe (FIG. 7).

## Example 2: *In vitro* and *in vivo* study of the effect of anti-γ-Sm scFv/hCPA and MTX-α-Phe on prostate cancer

In this study, effects of the pharmaceutical kit of the invention on prostate cancer were tested both *in vivo* and *in vitro*.

## Materials and Methods

**1. Reagents and cell lines:** Methotrexate (MTX) was purchased from No. 12 Pharmaceutical Factory of Shanghai. Two MTX-α-peptide derivatives, MTX-α-Phe and MTX-α-Arg, were synthesized by solid-phase synthesis. HPLC assay determined the sample as a single peak. Laser mass spectra found their molecular weights were consistent with theoretic values. CPA (Carboxypeptidase A) was a product of Sigma Chemicals (St. Louis, Mo, USA). A conjugate, γ-Sm-McAb-CP-A, was prepared by the inventor using the Melton method (Melton R. G, J. Immunol. Methods, 1993, 158:49). Anti-γ-Sm scFv/hCPA fusion protein was prepared as described in Example 1. The CPA enzyme activity of the conjugate was 20U/mg (CPA: McAb). Assessment of the biological activity of the fusion protein indicated that it has the activities of both anti-scFv and hCPA (See Example 1).

The human prostate cancer cell lines PC-3 (low metastatic) and PC-3m (high metastatic) were kindly provided by Department of Pathology, Beijing Medical University. The two cell lines were cultured in RPMI1640 complete medium at an atmosphere of 150ml/L $CO_2$ and digested with 2.5m/L trypsin for passage.

Tumor Model: BALB/C nude mice aged 4-6 weeks and weight at 16-20g were provided by the Experimental Animal Center of the Fourth Military Medical University (Xi'an, China) and bred in a sterile isolator. They were derma-inoculated with 0.1 ml of PC-3m suspension ($1 \times 10^7$ cells). The drugs were administrated when the volume of tumor was approximately 0.5 $cm^3$.

**2. *In vitro* cytotoxic assay of the fusion protein to PC cells**

**1) MTT assay:** PC-3 and PC-3m cells were seeded separately to a 96-well plate at $1 \times 10^4$cells/ml/well. MTX, MTX-$\alpha$-Phe, MTX-$\alpha$-Phe + CP-A, MTX-$\alpha$-Phe + fusion proteins and MTX-$\alpha$-Arg, MTX-$\alpha$-Arg + fusion proteins at different concentrations were also added into those wells. 3 parallel wells were set for each concentration. The cells were incubated in a $CO_2$ incubator for 48h. 20 $\mu$l of a solution of thiazole blue in PBS (5mg/ml) was added to each well and incubated at 37°C for 4h. The culture medium and free thiazole blue were discarded and 0.1ml of dimethylsulfoxide was added to each well. The absorbance at 405nm was measured and mortality rate was calculated.

**2) Colony formation assay.** The colony formation assay was performed using soft agar culture method. 9 different groups were included in the assay: ① MTX+PC cell, ② MTX-$\alpha$-Phe + PC cell, ③ fusion protein+PC cell, ④ MTX-$\alpha$-Phe + CP-A+PC cell, ⑤ MTX-$\alpha$-Phe + fusion protein + PC cell, ⑥ MTX-$\alpha$-Arg + PC cell, ⑦ MTX-$\alpha$-Arg + CP-A + PC cell, ⑧ MTX-$\alpha$-Arg + fusion protein +PC cell and ⑨ control group (PC cell only). All the tested prodrugs were used in three different concentrations, i.e. each of MTX, MTX-$\alpha$-Phe and MTX-$\alpha$-Arg was used at 0.4, 4, and 40$\mu$g/ml. CP-A was used in a volume of 10$\mu$l (2.5mU/$\mu$l) and fusion protein was used in a volume of 10$\mu$l (1mg/ml). The density of PC cell was 200/well. The plate was incubated in a $CO_2$ incubator at 37°C for 2 weeks. After incubation, PC cells were fixed by a mixture of acetic acid and ethanol (1:3) for 2h. Then microscopy was employed to calculate the percentage of colony formation.

**3) Kinetic effects of the fusion protein/prodrug on the growth cycle of PC cells:** PC-3 and PC-3m cells were added to several 10ml culture flasks($10^6$/ml). MTX, MTX-$\alpha$-Arg, MTX-$\alpha$-Phe + CP-A, MTX-$\alpha$-Phe + fusion protein at different concentrations were added to the flasks, respectively. The cells were collected at 6h, 16h and 24h after the addition of the tested drugs and washed twice with cold saline and then fixed with 950ml/L ethanol. The cells were stained by routine DNA staining method. Flow cytometry was employed in the kinetic analysis (FACS Profile II; Laser rate: 15mW; Green light filter: 488nm).

**3. *In vivo* study of tumor inhibition in animal models**

**1) Groups and treatments.** 56 nude mice carrying tumors were randomized into 7 groups: ① Control group, intravenously administered with 0.5ml of saline; ② Treatment Group I, intravenously administered with MTX (5mg/kg); ③ Treatment Group II, intravenously administered with MTX-$\alpha$-Phe (5.32mg/kg) ④ Treatment Group III, intravenously administered with 0.5ml of anti-$\gamma$-SmMcAb-CP-A; ⑤ Treatment Group IV, intravenously administered with 0.5ml of the fusion protein; ⑥ Treatment Group V, intravenously administered with 0.5ml of $\gamma$-SmMcAb-CP-A first and MTX-$\alpha$-Phe (5.32 mg/kg) 72h later (ADEPT-1); ⑦ Treatment Group VI, intravenously administered with 0.5ml of the fusion protein first and MTX-$\alpha$-Phe (5.32 mg/kg) 72h later (ADEPT-2).

**2) Observation Indexes**

***In vivo* distribution of the fusion protein:** The fusion protein was labeled by $^{125}$I and ECT was performed at 12h, 24h, 48, 72h and 96h after the intravenous injection to observe the localization of the antibodies. 2 nude mice in each group were killed and their tumor, brain, heart, liver, spleen, lung, kidney, stomach, small intestine, mesenteric lymph node, skeletal muscle, ovary and testicles were removed and weighed. One part of each organ and blood were used directly to measure the radioactivity and the rest part was homogenized with a NaCl solution (9g/L). Equal amount of trichloroacetic acid (200 ml/L) was mixed with each homogenate and the radioactivity of the pellet was measured. The content of the fusion protein in the tissue was calculated.

**Tumor-inhibition rate:** The major diameter and minor diameter of the tumor were measured every week after the administration of drugs. The mass of tumor ($m$ = 1/2 X major diameter X minor diameter$^2$) and tumor-inhibition rate (TIR) was thus calculated according the following formula.

$$\text{TIR} = \frac{\textit{Tumor mass of the control - Tumor mass of the experimental group}}{\textit{Tumor mass of the control}} \ X\ 100\%$$

**Cell cycle of tumor cells** is calculated by the formula: $t_c = [1g(1+G)/1g(m_2/m_1)] \times t$ (G: a component of the tumor growth group; $m_2$, $m_1$ : tumor mass of a given component of the group observed at different time points; $t$: interval between observations).

**Survival time of the nude mice** refers to the period from the beginning of the treatment till natural death.

**Histopathologic observation** includes gross specimen examination and microscopic examination. Samples of tumor and organs were fixed with 100ml/L formalin. Pathologic section was stained by and then underwent microscopy observation.

## RESULTS

### *In vitro* cytotoxicity assay

1) **Cytotoxicity assay:** The lethal curves of different concentrations of MTX, MTX-$\alpha$-Phe, MTX-$\alpha$-Phe+CP-A, MTX-$\alpha$-Phe+fusion protein and MTX-$\alpha$-Arg indicated that prodrugs MTX-$\alpha$-Phe and MTX-$\alpha$-Arg have no lethal effect to PC cells whereas the fusion protein has strong cytotoxic effect to PC cells (FIG. 8). The cytotoxicity of fusion protein was equal to that of MTX but approximately 1,000 times higher than that of the prodrug., The $IC_{50}$ of free MTX produced by the fusion protein was $4.5 \times 10^{-6}$ mol/L. However, the prodrug didn't have any significant toxicity on PC cells, with an $IC_{50}$ of $7.2 \times 10^{-2}$ mol/L.

2) **Colony formation assay:** Neither MTX-$\alpha$-Phe nor MTX-$\alpha$-Arg could inhibit the growth of PC cells. Their colony-formation rates were 88% and 91% respectively, not significantly different from that of the control (P>0.05). In contrast, MTX, MTX-$\alpha$-Phe+CP-A, and MTX-$\alpha$-Phe+fusion protein all had a considerably inhibitory effect and none of these groups had any colony formed (P<0.001). The fusion protein could inhibit the growth of PC cells and had a colony-formation rate of 68%, different from the control (P<0.01).

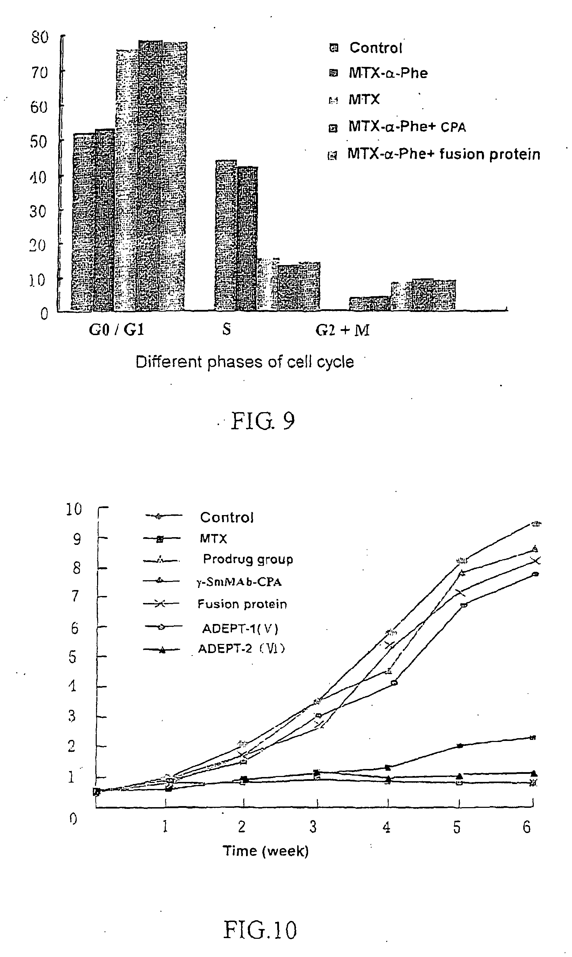

3) **Cell cycle analysis:** The percentage of cells in $G_1/G_0$ phase, $G_2$+M phase was increased significantly in PC cells treated with MTX-$\alpha$-Phe+fusion protein ($t$ =15.04 和 2.76, P<0.01 and P<0.05) whereas that of S phase declined significantly ($t$=17.85, P<0.01). Similar as treated with MTX-$\alpha$-Phe+ CP-A and MTX, the cell proliferation index was decreased significantly and apoptosis was found in MTX-$\alpha$-Phe+fusion protein treated cells. Cells treated with prodrug MTX-$\alpha$-Phe did not show any significant kinetic change (FIG. 9).

### 2. *In vivo* experiments in the animal models

1) *In vivo* **distribution of the fusion protein:** The distribution of $^{125}$I-labeled fusion protein among blood, tumor and other organs at 12h, 24h, 48h, 72h and 96h after the injection of fusion protein was shown in Table 1. ECT scanning indicated that the isotope was aggregated significantly in tumor sites. The aggregation was highest at 72h with a T/NT value of 12.65 at that time point. The results indicated that the antibody can efficiently target the fusion protein into tumor site specifically.

Table 1  Distribution of $^{125}$I-labeled fusion protein among the organs at different time points after i.v. injection

| | % ID / g | | | | | | | | | | | | | | |
|---|---|---|---|---|---|---|---|---|---|---|---|---|---|---|---|
| time (h) | Liver | Stomach | Kidney | Lung | heart | Muscle | Brain | Lymph Node | Intestine | Bladder | Thyroid | Testis | Ovary | Blood | Tumor |
| 12 | 5.06 | 5.00 | 4.94 | 5.78 | 4.82 | 2.28 | 0.82 | 2.57 | 2.38 | 4.15 | 5.01 | 0.78 | 0.73 | 14.29 | 5.78 |
| 24 | 7.06 | 7.74 | 6.58 | 8.26 | 5.67 | 2.34 | 1.09 | 2.41 | 2.13 | 4.62 | 4.71 | 0.95 | 0.97 | 12.12 | 9.42 |
| 48 | 7.51 | 7.56 | 6.84 | 8.79 | 6.21 | 2.41 | 1.13 | 2.32 | 1.87 | 4.81 | 4.12 | 1.32 | 1.27 | 9.53 | 12.89 |
| 72 | 9.21 | 8.54 | 7.11 | 8.02 | 6.85 | 2.52 | 1.23 | 2.30 | 1.50 | 4.88 | 3.01 | 1.59 | 1.56 | 7.73 | 15.96 |
| 96 | 8.83 | 8.72 | 7.14 | 9.27 | 7.17 | 2.78 | 1.28 | 2.27 | 1.43 | 4.02 | 2.36 | 1.75 | 1.69 | 7.07 | 14.08 |

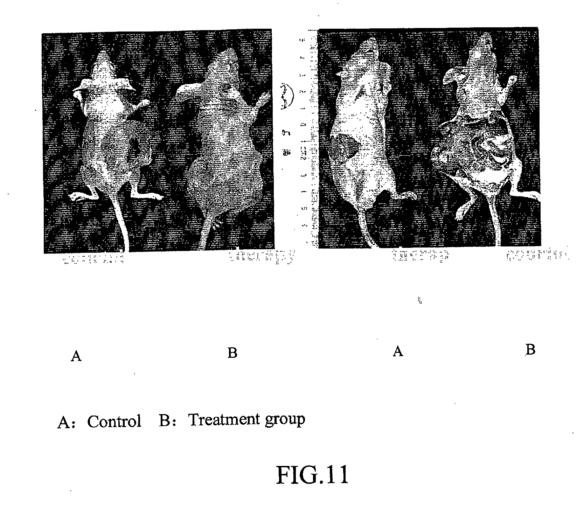

**2) Dynamic observation of the tumor mass:** In the 1st week, no significant difference of the mass of the tumors was observed among the groups. From the 2nd week on, the growth of tumors was decreased in treatment groups I, V and VI. By the 6th week, the volume of tumors in these three groups had become significantly smaller than that in other groups. In groups V and VI (the ADEPT groups), tumors in 12 mice decreased significantly in size and the tumors almost completely disappeared in 4 mice. Tumor-inhibition rate of Group V and group VI was significantly higher than that of other groups (P<0.001) and there was no significant difference between these two groups. Tumors in groups II, III, IV were small compared with that of the control but the difference was not significant (P>0.05). The tumors in the control group grew so fast that by the latter phase, some tumors became diabrotic (FIG. 11).

**3) A comparison between growth of tumor cells and survival time of the nude mice:** The tumor in the control group grew faster than those in treatment groups. In control group, tumor cell growth cycle was 1.5±0.1, and 3 mice had extensive metastasis in the abdominal cavity, serious ascitic fluid and cachexia. The survival time of these 3 mice was 28±11.7 days. There was no significant difference between treatment groups II, III and IV with control group (P<0.05). Their cell growth cycles were 1.7±0.2, 2.7±0.2 and 1.9±0.1, respectively, while the average survival time were 31±12.2, 39±15.8, and 34±14.1 days respectively. Mice treated with MTX alone had poor living quality, although its tumor growth was inhibited. Their whole blood count was significantly lower than that of the ADEPT groups (groups V and VI) (P<0.01). The cell growth cycle and lifespan of the mice of this group (MTX alone) were 2.5±0.2 and 67±13.8 days respectively. In contrast, the cell growth cycles of the ADEPT groups were the longest, 3.7±0.2 and 3.4±0.5 respectively and their mice had an average lifespan of 91±13.9 and 89±12.6 days respectively.

**4) Histopathologic examination:** After 5 weeks of treatment, gross specimen examination found a large area of necrosis in the tumors of groups I, V and VI. The cut of the tumor looked yellow and white and its tissues were slack and fragile. There were a few envelope and tumor tissues remaining on the peripheral areas. Microscopy examination found some sheet-shaped calcification scattered in the necrotic tumor tissues and only the contour of the cancer histolysis remained. In contrast, the tumors in the control group and groups II, III and IV showed characteristics typical of adenocarcinoma, active cancer cell growth and extensive cancer tissue infiltration and metastasis in the abdominal cavity of the mice.

**Claims**

1. A pharmaceutical kit for treating prostate cancer via the antibody-directed enzyme prodrug therapy, comprising several discrete containers containing, respectively, a fusion protein of a human γ-seminoprotein single-chain antibody and human carboxypeptidase A (anti-γ-Sm scFv/hCPA fusion protein), a prodrug which is a methotrexate-α-peptide and a pharmaceutically acceptable carrier, wherein the anti-γ-Sm scFv/hCPA fusion protein is administered at least 72 hours before the prodrug is administered.

2. The pharmaceutical kit according to claim 1, wherein said prodrug is methotrexate-α-phe.

3. A fusion protein of a human γ-seminoprotein single-chain antibody and human carboxypeptidase A, comprising an amino acid sequence as shown in FIG. 4.

4. A fusion gene encoding the fusion protein according to claim 3, comprising a nucleotide sequence as shown in FIG. 3.

5. Use of the pharmaceutical kit according to claim 1 in to the treatment of prostate cancer.

FIG. 1

1: ScFv PCR product;

2: hCPA PCR product;

3: ScFv /hCPA SOE product;

M: Molecular weight marker λ DNA/EcoRI+Hind III
   From top to bottom: 21227bp, 5148bp, 4268bp, 3530bp,
   2027bp, 1904bp, 1584bp, 1375bp, 947bp, 831bp, and 564bp

# FIG. 2

EcoR I |——————— ScFv ——————→

GAA TTC ATG CAG GTC CAA CTG CAG GAG TCT GGA CCT GGC CTG GTG GCG CCC TCA CAG AAC

CTG TCC ATC ACT TGC ACT GTC TCT GGG TTT TCA TTA ACC AGC TAT GGT GTA CAC TGG GTT

CGC CAG CCT CCA GGA AAG GGT CTG GAA TGG CTG GGG GTA ATA TGG ACT GGT AGA AGC ACA

ACT TAT AAT TCG GCT CTC ATG TCC AGA CTG AGC ATC AGC AAA GAC AAC TCC GAG AGC CAA

GTT TTC TTA AAA ATG AAC AGT CTG CAG ACT GAT GAC ACA GCC ATT TAC TAC TGT GGC AGA

GGG GGT CTG ATT ACG TCC TTT GCT ATG GAC TAC TGG GGC CAA GGG ACC ACG GTC ACC GTC

TCC TCA GAG CTC GGT GGC GGC GGT TCA GGC GGA GGT GGC TCT GGC GGT GGC GGA TCC GAC

ATT GTG ATG ACC CAG TCT CCA TCC TCC CTG TCT GCC TCT CTG GGA GAC AGA GTC ACC ATC

AGT TGC AGG GCA AAT CAG GAC ATT AGC AAT TAT TTA AAC TGG TAT CAG CAG AAA CCA GAT

GGA ACT GCT AAA CTC CTG ATC TAC TAC ACA TCA AGA TTA CAC TCA GGG GTC CCA TCA AGG

TTC AGT GGC AGT GGG TCT GGA ACA GAT TAT TCT CTC ACC ATT AGC AAC CTG GAC CAA GAA

GAT TTT GCC ACT TAC TTT TGC CAA CAG GGT AAT ACG CTT CCG TGG ACG TTC GGT GGA GGG

ACC AAG CTG GAG ATC AAA CGT GGT AGC GGC GGT TCT GGT AGG CTC ATC CTG CCT GTG GGT

←—— ScFv ——————‖——— linker ———————‖——— hCPA —→

TTG ATT GCT ACC ACT CTT GCA ATT GCT CCT GTC CGC TTT GAC AGG GAG AAG GTG TTC CGC

GTG AAG CCC CAG GAT GAA AAA CAA GCA GAC ATC ATA AAG GAC TTG GCC AAA ACC AAT GAG

CTT GAC TTC TGG TAT CCA GGT GCC ACC CAC CAC GTA GCT GCT AAT ATG ATG GTG GAT TTC

CGA GTT AGT GAGAAG GAA TCC CAA GCC ATC AGT CTG CCT TGA TCA AAA TAA AAT GCAC

TAT GAA ATC TTG ATT CAT GAT CTA CAA GAA GAG ATT GAG AAA CAG TTT GAT GTT AAA GAA

GAT ATC CCA GGC AGG CAC AGC TAC GCA AAA TAC AAT AAT TGG GAA AAG ATT GTG GCT TGG

ACT GAA AAG ATG ATG GAT AAG TAT CCT GAA ATG GTC TCT CGT ATT AAA ATT GGA TCT ACT

GTT GAA GAT AAT CCA CTA TAT GTT CTG AAG ATT GGG GAA AAG AAT GAA AGA AGA AAG GCT

ATT TTT ATG GAT TGT GGC ATT CAC GCA CGA GAA TGG GTC TCC CCA GCA TTC TGC CAG TGG

TTT GTC TAT CAG GCA ACC AAA ACT TAT GGG AGA AAC AAA ATT ATG ACC AAA CTC TTG GAC

CGA ATG AAT TTT TAC ATT CTT CCT GTG TTC AAT GTT GAT GGA TAT ATT TGG TCA TGG ACA

AAG AAC CGC ATG TGG AGA AAA AAT CGT TCC AAG AAC CAA AAC TCC AAA TGC ATC GGC ACT

GAC CTC AAC AGG AAT TTT AAT GCT TCA TGG AAC TCC ATT CCT AAC ACC AAT GAC CCA TGT

GCA GAT AAC TAT CGG GGC TCT GCA CCA GAG TCC GAG AAA GAG ACG AAA GCT GTC ACT AAT

TTC ATT AGA AGC CAC CTG AAT GAA ATC AAG GTT TAC ATC ACC TTC CAT TCC TAC TCC CAG

ATG CTA TTG TTT CCC TAT GGA TAT ACA TCA AAA CTG CCA CCT AAC CAT GAG GAC TTG GCC

AAA GTT GCA AAG ATT GGC ACT GAT GTT CTA TCA ACT CGA TAT GAA ACC CGC TAC ATC TAT

GGC CCA ATA GAA TCA ACA ATT TAC CCG ATA TCA GGT TCT TCT TTA GAC TGG GCT TAT GAC

CTG GGC ATC AAA CAC ACA TTT GCC TTT GAG CTC CGA GAT AAA GGC AAA TTT GGT TTT CTC

CTT CCA GAA TCC CGG ATA AAG CCA ACG TGC AGA GAG ACC ATG CTA GCT GTC AAA TTT ATT

GCC AAG TAT ATC CTC AAG CAT ACT TCC TAA AGA ACT GGT CGA C

←—————————— hCPA ——————————| Sal I

# FIG. 3

MQVQLQESGPGLVAPSQNLSITCTVSGFSLTSYGVHWVRQPPGKGLEWL

GVIWTGRSTTYNSALMSRLSISKDNSESQVFLKMNSLQTDDTAIYYCGR

GGLITSFAMDYWGQGTTVTVSSELGGGGSGGGGSGGGGSDIVMTQSPSS

LSASLGDRVTISCRANQDISNYLNWYQQKPDGTAKLLIYYTSRLHSGVPS

RFSGSGSGTDYSLTISNLNQEDFATYFCQQGNTLPWTFGGGTKLEIKRGC

GGSGRLILPVGLIATTLAIAPVRFDREKVFRVKPQDEKQADIIKDLAKTNE

LDFWYPGATHHVAANMMVDFRVSEKESQAIQSALDQNKMHYEILIHDL

QEEIEKQFDVKEDIPGRHSYAKYNNWEKIVAWTEKMMDKYPEMVSRIKI

GSTVEDNPLYVLKIGEKNERRKAIFMDCGIHAREWVSPAFCQWFVYQAT

KTYGRNKIMTKLLDRMNFYILPVFNVDGYIWSWTKNRMWRKNRSKNQ

NSKCIGTDLNRNFNASWNSIPNTNDPCADNYRGSAPESEKETKAVTNFIR

SHLNEIKVYITFHSYSQMLLFPYGYTSKLPPNHEDLAKVAKIGTDVLSTR

YETRYIYGPIESTIYPISGSSLDWAYDLGIKHTFAFELRDKGKFGFLLPESRI

KPTCRETMLAVKFIAKYILKHTS

FIG. 4

1, 2, 3, 4: Induced expression products;
5, 6: JM109 strains (not induced);
M: Molecular weight marker, from top to bottom as 97400, 66200, 43000, 31000, 20100, and 14400

FIG. 5

A: Control McAb;    B: Fusion protein + γ-Sm McAb;    C: γ-Sm McAb

FIG. 6

FIG. 7

FIG. 8

FIG. 9

FIG.10

A B A B

A: Control B: Treatment group

FIG.11

## INTERNATIONAL SEARCH REPORT

| | |
|---|---|
| Inte nal application No. | PCT/CN01/00924 |

**A. CLASSIFICATION OF SUBJECT MATTER**

IPC$^7$  A61K39/395, A61P35/00

According to International Patent Classification(IPC) or to both national classification and IPC

**B. FIELDS SEARCHED**

Minimum documentation searched(classification system followed by classification symbols)

IPC$^7$  A61K39/395, A61P35/00

Documentation searched other than minimum documentation to the extent that such documents are included in the field searched

Electronic data base consulted during the international search(name of data base and, where practicable, search terms used)

CNPAT, EPOQUE(WPI), NCBI

**C. DOCUMENTS CONSIDERED TO BE RELEVANT**

| Category* | Citation of document, with indication, where appropriate, of the relevant passages | Relevant claim No. |
|---|---|---|
| A | WO, A2, 9939740, ((INEX-N) INEX PHARM CORP), 12, August, 1999, see the abstract. | 1—5 |
| A | WO, A1, 9835988, ((ZENE ) ZENECA LTD), 20, August, 1998, see the abstract. | 1—5 |
| A | WO, A1, 9835982, ((ZENE ) ZENECA LTD), 20, August, 1998, see the abstract. | 1—5 |
| A | WO, A1, 9707118, ( (CANC-N) CANCER RES CAMPAIGN TECHNOLOGY, et al), 27, February, 1997, see the abstract. | 1—5 |
| A | Acta Pharmaceutica Sinica, vol. 32, no.2, 1997, SHI Putao, "the solid phase synthesis of methotrexate-α-peptides", p106-109, see entire document. | 1—5 |

☐ Further documents are listed in the continuation of Box C.   ☒ See patent family annex.

| | |
|---|---|
| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason(as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 18 January 2002(18.01.2002) | 31. JAN 2002 (31.01.02) |

| Name and mailing address of the ISA/<br>The Chinese Patent Office<br>6, Xitucheng Road, Haidian District,<br>Beijing, 100088, China<br>Facsimile No.  86-10-62019451 | Authorized officer<br>WEI chunbao<br>Telephone No.  86-10-62093435 |

Form PCT/ISA/210(second sheet)(July 1992)

**INTERNATIONAL SEARCH REPORT**

Information on patent family members

| Intern: pplication No. |
|---|
| PCT/CN01/00924 |

| Patent document cited in search report | Publication date | Patent family members | Publication date |
|---|---|---|---|
| WO-A2-9939740 | 12-08-1999 | AU-A-2405699<br>EP-A2-1053022 | 23-08-1999<br>22-11-2000 |
| WO-A1-9835988 | 20-08-1998 | AU-A-6000698 | 08-09-1998 |
| WO-A1-9835982 | 20-08-1998 | ZA-A-9801222<br>AU-A-6000498 | 28-10-1998<br>08-09-1998 |
| WO-A1-9707118 | 27-02-1997 | US-A-6124310<br>AU-A-6711096<br>NZ-A-315411<br>JP-T-11511163T<br>EP-A1-0853623 | 26-09-2000<br>12-03-1997<br>28-01-2000<br>28-09-1999<br>22-07-1998 |

Form PCT/ISA/210(patent family annex)(July 1992)